# EUROPEAN PATENT SPECIFICATION

(11) **EP 3 615 943 B1**
(45) Date of publication and mention of the grant of the patent: **31.03.2021**
(21) Application number: 18725302.6
(22) Date of filing: 26.04.2018
(51) Int. Cl.: G01N 33/68, A61K 31/19, A61K 31/385

(54) **KIT FOR THE ASSESSMENT OF ENDOMETRIAL RECEPTIVITY**
KIT ZUR BEURTEILUNG DER ENDOMETRIALEN AUFNAHME
TROUSSE POUR L'ÉVALUATION DE LA RÉCEPTIVITÉ DE L'ENDOMÈTRE

(30) Priority: 27.04.2017 IT 201700045856
(43) Date of publication of application: 04.03.2020
(73) Proprietor: Molipharma S.r.l., 86100 Campobasso (IT)
(72) Inventor: SCAMBIA, Giovanni, 86100 Campobasso (CB) (IT); DI SIMONE, Nicoletta, 86100 Campobasso (CB) (IT); D'IPPOLITO, Silvia, 86100 Campobasso (CB) (IT)
(74) Representative: Predazzi, Valentina
(86) International application number: PCT/IB2018/052884
(87) International publication number: WO 2018/198054

(56) References cited:
- WO-A1-2011/147976
- WIZNITZER ET AL: "Lipoic acid prevention of neural tube defects in offspring of rats with streptozocin-induced diabetes", AMERICAN JOURNAL OF OBSTETRICS & GYNECO, MOSBY, ST LOUIS, MO, US, vol. 180, no. 1, 1 January 1999 (1999-01-01), pages 188-193, XP005142478, ISSN: 0002-9378, DOI: 10.1016/S0002-9378(99)70173-0
- STORTONI PIERGIORGIO ET AL: "Placental thrombomodulin expression in recurrent miscarriage", REPRODUCTIVE BIOLOGY AND ENDOCRINOLOGY, BIOMED CENTRAL LTD, GB, vol. 8, no. 1, 5 January 2010 (2010-01-05) , page 1, XP021068207, ISSN: 1477-7827, DOI: 10.1186/1477-7827-8-1
- DIAZ-GIMENO P ET AL: "A genomic diagnostic tool for human endometrial receptivity based on the transcriptomic signature", FERTILITY AND STERILITY, ELSEVIER SCIENCE INC, NEW YORK, NY, USA, vol. 95, no. 1, 1 January 2011 (2011-01-01), pages 50-60.e15, XP027563196, ISSN: 0015-0282 [retrieved on 2010-07-08]
- D'IPPOLITO SILVIA ET AL: "Inflammosome in the human endometrium: further step in the evaluation of the "maternal side"", FERTILITY AND STERILITY, ELSEVIER SCIENCE INC, NEW YORK, NY, USA, vol. 105, no. 1, 30 October 2015 (2015-10-30), page 111, XP029375305, ISSN: 0015-0282, DOI: 10.1016/J.FERTNSTERT.2015.09.027
- MONASTRA GIOVANNI ET AL: "Immunomodulatory activities of alpha lipoicacid with a special focus on its efficacy in preventing miscarriage", EXPERT OPINION ON DRUG DELIVERY, INFORMA HEALTHCARE, GB, vol. 13, no. 12, 30 November 2016 (2016-11-30), pages 1695-1708, XP009501283, ISSN: 1742-5247, DOI: 10.1080/17425247.2016.1200556 [retrieved on 2016-06-24]

## Description

The present description relates to a method and a diagnostic kit for analysing the inflammatory state and endometrial receptivity in women suffering from unexplained spontaneous recurrent abortion and/or infertility, and to alpha-lipoic acid for use in the treatment or assistance in the treatment of endometrial inflammation. The invention is based on analysis of the ratio between the levels of expression of the NALP-3 and thrombomodulin proteins in the endometrium. A ratio of greater than 1 is indicative of an increased condition of endometrial inflammation compared to a fertile subject and is associated with reduced endometrial receptivity.

### PRIOR ART

Spontaneous recurrent abortion (SRA) and infertility are complications that affect 15-20% of women of reproductive age. Nowadays, according to research acknowledged in the field of evidence-based medicine, a high percentage of cases, equal to approximately 30-35%, are unexplained. An adequate implantation process requires complex interaction between embryo and the woman's endometrium, which, in order to promote the process of adhesion and implantation of the embryo, must undergo suitable changes aimed at increasing its receptivity.

It has become clear from numerous items of scientific evidence that increased endometrial levels of pro- and anti-inflammatory factors can be underlying mechanisms of SRA and/or infertility. A disadvantageous endometrial cytokine state could be a non-specific consequence of the activation of a natural immunity by exogenous and/or endogenous stimuli.

One of the components of natural immunity is inflammasome, which is a cytoplasmic multiprotein complex with an important "sentry" role, guarding against infectious agents (viruses, bacteria) and non-infectious agents (damaged cells, cell fragments). It promotes activation of the inflammatory processes by way of activation of caspase-1, which, for its part, determines the proteolytic cleavage of the precursors of the interleukins (IL)-1β and -18. The consequent increase in the respective active forms triggers an inflammatory response, the ultimate objective of which is to eliminate pathogenic agents or block their pathogenicity.

It has recently been demonstrated that the activation/expression of inflammasome can be modulated by thrombomodulin (TM), which is a membrane protein expressed by different cell types, such as endothelial cells, syncytiotrophoblast, keratinocytes, intestinal epithelial cells, and endometrial stromal cells. It develops an important anticoagulant action, being physiologically involved in the final activation of the C protein and also involved in the processes for controlling local inflammation. Its own expression can be inhibited in a time- and dose-dependent manner in the presence of excessive inflammation, as observed in animal models of sepsis induced by lipopolysaccharides. It has been found from previous *in vivo* studies that the pregnancy of knock-out mice by way of TM is interrupted prematurely by means of mechanisms not attributable to thrombotic phenomena, suggesting that TM plays a fundamental role in the process of placentation, which is independent of its known anticoagulant activity. In conformity with such observations, a significant reduction in the expression of TM in the placenta tissue of women suffering from SRA has been demonstrated. On the whole, such evidence strongly supports the role of TM in the preservation of the initial pregnancy by means of mechanisms not related to the known antithrombotic action.

In a previous ex *vivo* study (→ Ippolito S, Tersigni C, Marana R, Di Nicuolo F, Gaglione R, Rossi ED, Castellani R, Scambia G, Di Simone N. Inflammosome in the human endometrium: further step in the evaluation of the "maternal side". Fertil Steril. 2016;105:111-8.e1-4.) the authors of the invention demonstrated the endometrial expression of two main components of inflammasome (NALP-3 and ASC) and observed an increase in the activation of NALP-3 and an increase in the secretion of IL-1β and IL-18 in endometrial biopsies of women with a history of idiopathic SRA, compared with a group of fertile women (control), in the absence of vaginal and/or endometrial infections and with histopathological pictures suggestive of chronic endometritis. This observation made it possible to suggest manipulated activation of inflammasome, caused by non-specific stimuli (currently undetermined), as one of the molecular mechanisms that can compromise endometrial receptivity and consequently determine a negative obstetric outcome.

The study of endometrial receptivity in women suffering from unexplained SRA and/or infertility still remains complex and is difficult to assess. Diagnostic hysteroscopy makes it possible to perform a morphological assessment of the endometrial cavity and, when combined with immunohistochemistry, to identify the pictures with chronic endometritis, a pathological condition encountered in approximately 25-30% of women suffering from SRA. Immunohistochemistry, however, has numerous limitations: although it has increased specificity, up to 93%, it has a low sensitivity, which does not exceed 30% and is heavily operator-dependent; its execution is time-consuming (each biopsy is first fixed in formalin, processed, and lastly enclosed in paraffin) and requires the input of more professional individuals (technicians, biologists specialised in histopathology, and skilled pathologists and experts in the tissue to be studied). This type of diagnosis normally singles out up to 15-20% of cases of endometrial inflammation and thus has a very low sensitivity threshold, and therefore the remaining cases of endometrial inflammation lie beyond the grasp of this type of diagnosis.

A diagnostic method called the Endometrial Receptivity Analysis (ERA) Test is currently commercially available. It is based on a microarray analysis of the endometrial tissue able to analyse the levels of expression of 238 genes linked to the state of endometrial receptivity. In reality, the gene expression expresses the potential of a specific tissue and not necessarily protein expression; thus, the applicability of this method is severely reduced insofar as it does not provide information regarding the actual state of endometrial inflammation.

Given these limitations of the various methods that can be used, the introduction of new diagnostic methods and kits useful in the assessment of the actual inflammatory state of the endometrium that give an objective result, not dependent on the operator, and which are easily executed and can be quickly performed is desirable.

### SUMMARY OF THE INVENTION

The authors of the present invention have discovered, by means of an ex *vivo* study performed on endometrial biopsies obtained from women suffering from idiopathic SRA, that during the "implantation window" there is increased activation of the components of inflammasome (NALP-3 and ASC) and therefore a rise in the production of pro-inflammatory cytokines IL-1β and IL-18. Such activation is not related to the presence of an immunohistochemical picture of chronic endometritis, suggesting that this determination provides more specific information regarding the endometrial condition unfavourable for implantation and thus reduced endometrial receptivity. The inventors also observed that women suffering from SRA have significantly reduced endometrial TM levels compared to fertile women (control). In particular, the authors of the invention have discovered that the presence at endometrial level of an altered ratio between the NALP-3/TM levels of expression makes it possible to obtain a more specific diagnosis of the endometrial state, particularly in women suffering from unexplained SRA or infertility who have a normal report in the diagnostic hysteroscopy and/or standard immunohistochemical exam. Thus, the authors have developed a diagnostic method based on endometrial analysis of the levels of such proteins which overcomes the limitations associated with immunohistochemical methodology. This method is easily executed, is cost-effective, is independent of the subjective assessment of an expert pathologist in the field of immunohistochemistry, and makes it possible to obtain a result/report within a relatively quick period of time, not requiring excessive handling of the sample.

The present invention therefore relates to the identification of new and more specific diagnostic markers for assessing the endometrial inflammatory state, kits that make it possible to study said state, and new diagnostic methods which, with a low level of invasiveness and low implementation costs, are able to provide information relating to the state of receptivity of the endometrium with increased sensitivity and specificity.

In particular, the present disclosure relates to: an *ex vivo* method for the analysis of a patient's endometrial receptivity comprising the following steps:
a. assessing the expression levels of the NALP-3 and thrombomodulin (TM) proteins with the same analytical method, in an endometrial biopsy sample and/or in an endometrial fluid sample and/or in a menstrual blood sample of said patient;
b. calculating the ratio between the expression level of the NALP-3 and TM proteins [NALP-3 expression/TM expression] wherein, when said value is greater than 1, it is a marker of inflammatory alteration of the endometrium of said patient and of an altered endometrial receptivity with respect to a healthy endometrium;
a kit for the analysis of endometrial receptivity comprising:
a. reagents for the assessment of NALP-3 and thrombomodulin (TM) expression and, optionally,
b. reagents for the assessment of the expression of caspase-1 and/or IL-18 and IL-1β and control samples representative of caspase-1, IL-18 and IL-1β expression in healthy patients;
   a support for ELISA or Multiplex immunoenzymatic assay comprising wells coated with anti-NALP-3 antibodies and anti-TM antibodies suitably arranged,
   a use of said kit or said support for the analysis of a patient's endometrial receptivity;
   a therapeutic treatment for the treatment of SRA comprising the analysis of endometrial receptivity by the method described above and suitable therapy in order to restore the correct endometrial state in the patients suffering from reduced endometrial receptivity compared to a healthy patient,
   alpha-lipoic acid for use in a therapeutic treatment of SRA optionally in combination with other active ingredients.

### DETAILED DESCRIPTION OF THE FIGURES

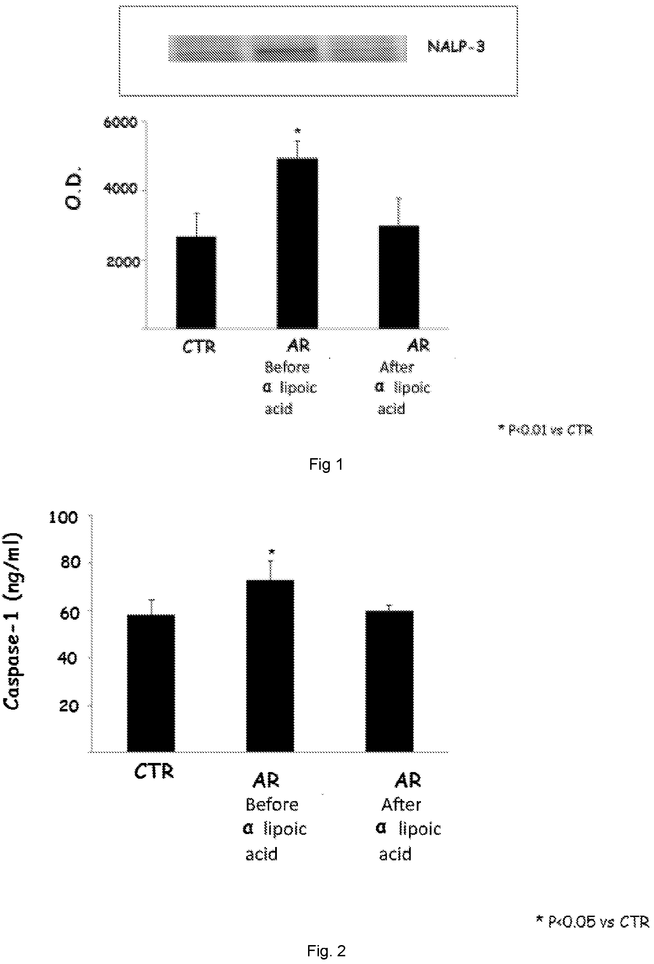

Figure 1 shows the concentration of NALP-3 (western blot) in endometrial biopsies obtained from unexplained polyabortive patients (AR) before and after treatment with alpha-lipoic acid compared to a control patient group (CTR) with previous physiological pregnancies.
Figure 2 shows the concentration of IL-1beta in endometrial biopsies obtained from unexplained polyabortive patients (AR) before and after treatment with alpha-lipoic acid compared to a control patient group (CTR) with previous physiological pregnancies.

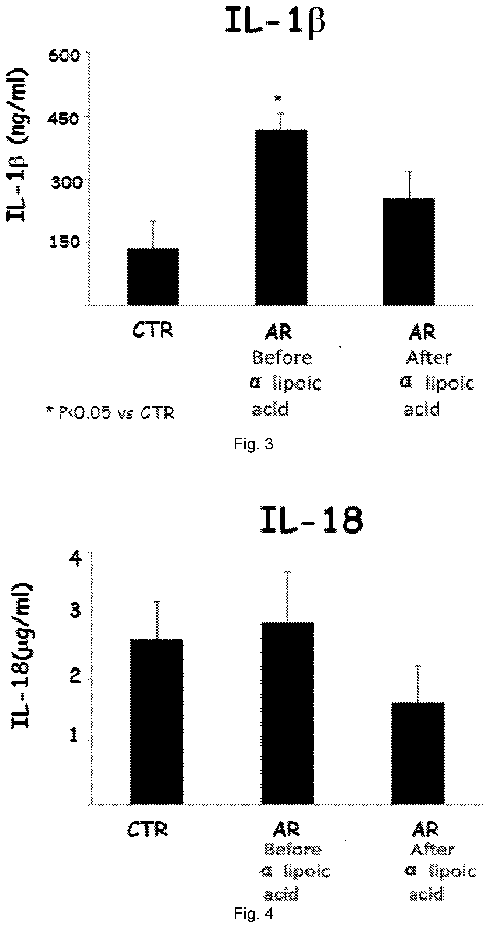

Figure 3 shows the concentration of IL-18 in endometrial biopsies obtained from unexplained polyabortive patients (AR) before and after treatment with alpha-lipoic acid compared to a control patient group (CTR) with previous physiological pregnancies.
Figure 4 shows the concentration of caspase 1 in endometrial biopsies obtained from unexplained polyabortive patients before and after treatment with alpha-lipoic acid compared to a control patient group (CTR) with previous physiological pregnancies (with regard to caspase 1, in general, the authors observed an overlap between the development of the expression of said caspase and the development of the expression of thrombomodulin.
Figure 5 shows the comparison between the expression values of NALP-3 calculated in the control sample compared to the sample of women suffering from SRA.
Figure 6 shows the comparison between the expression values of TM calculated in the control sample compared to the sample of women suffering from SRA.
Figure 7 shows the comparison between the RIT values (NALP-3/TM ratio) calculated in the control sample compared to the sample of women suffering from SRA.
Figure 8 shows the comparison between the expression values of caspase 1 calculated in the control sample compared to the sample of women suffering from SRA.

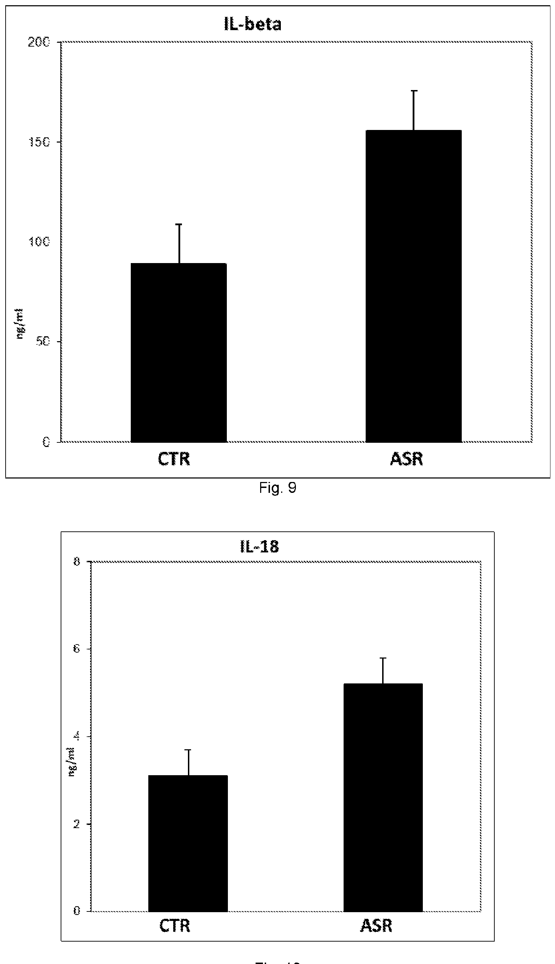

Figure 9 shows the comparison between the expression values of IL-beta calculated in the control sample compared to the sample of women suffering from SRA.
Figure 10 shows the comparison between the expression values of IL-18 calculated in the control sample compared to the sample of women suffering from SRA.

### GLOSSARY

NAPL-3 or NALP3 (acronym for NACHT, LRR and PYD domains-containing protein 3) is a protein encoded by the human NPLR3 gene, which maps on the long arm of chromosome 1. The protein is expressed predominantly in macrophages and as a component of inflammasome.
Some known further names for this protein are: AGTAVPRL, All, AVP, C1orf7, CIAS1, CLR1.1, FCAS, FCAS1, FCU, MWS, NALP3, PYPAF1, NLR family, pyrin domain containing 3, NLR family pyrin domain containing 3.

Thrombomodulin (TM or CD 141 or BDCA-3) is an integral membrane protein expressed on the surface of the endothelial cells and in particular on the stromal endometrial cells. In humans, thrombomodulin is encoded in the gene called THBD. It is an important mediator in the activation of active protein C and has a crucial role in local control of inflammation.

INFLAMMASOME: multiprotein cytoplasmic complex that can be activated in response to intra- and extracellular "danger" signals. Once activated, inflammasome triggers an inflammatory response to the activation of caspase-1 and consequently the release of interleukins (IL)-1β and -18.

unexplained SRA (spontaneous recurrent abortion) or infertility: condition in which, after having ruled out all of the known causes of SRA or infertility, it is not possible to identify an underlying cause. Endometrial receptivity: the term within the present description has the meaning commonly used in literature and indicates the receptivity of the endometrium to receive implantation of an embryo. "Normal" endometrial receptivity, in the present description, means the receptivity presented by a control population, formed of women with previous physiological pregnancies, and an endometrial receptivity different from the "normal" receptivity, as defined above, is defined here as an "altered" endometrial receptivity.

### DETAILED DESCRIPTION OF THE INVENTION

Implantation is a complex process during which the embryo adheres to the endometrium and draws therefrom the nutrients useful for its development. This process occurs when the embryo reaches the stage of maturation called the blastocyst stage.

Implantation consists of three phases (which last for a total of approximately 7 days): in the first phase (apposition) the embryo must rest against the endometrium, and in the second phase (adhesion) it must make significant bonds with the endometrium (must "latch on"), and in the final phase (invasion) it must penetrate within the endometrium itself in order to be completely accepted. Implantation finishes around day 14 after fertilisation (or approximately day 28 of the menstrual cycle). If implantation is successful, the pregnancy (initiated from approximately day 14) can continue.

In all of these phases both the embryo and the endometrium play an active role: both secrete numerous substances defining a real "biochemical dialogue" therebetween. The efficacy of this "dialogue" is fundamental for the success of the implantation. Thus, both suitable development of the embryo and adequate endometrial "receptivity" are fundamental for implantation to occur correctly. Adequate endometrial receptivity is a condition that is *necessary* for the initiation and continuation of the pregnancy. As known in the literature, between 30 and 50% of failed pregnancies can be attributed to unsuccessful implantation.

Currently, unfortunately, in approximately 30-40% of cases of spontaneous abortion and/or infertility, it is not possible to determine the reasons as to why pregnancy has failed or why it continues to fail.

As mentioned above, the authors of the invention have previously observed that, in women suffering from unexplained SRA, there is an increased activation of the components of inflammasome in the endometrium during the period of the implantation window not linked to the presence of an immunohistochemical picture of chronic endometritis, thus demonstrating the importance of finding new diagnostic methods and more specific endometrial markers. The ultimate objective is that of enabling the identification of a personalised therapy tailored to each individual patient, so as to allow an improvement in the endometrial receptivity.

The authors of the present invention have surprisingly discovered that the value of the ratio [expression value of NALP-3/expression value of TM] makes it possible to identify 70-80% of cases of endometrial inflammation. A value greater than 1, in fact, is said to be always associated with endometrial inflammation and with a subsequent reduction in endometrial receptivity.

In the case of values close to or equal to 1, the last assessment of the expression levels of caspase 1 and one or more inflammatory cytokines can make it possible to further improve the assessment and to identify up to 90% of cases of reduced endometrial receptivity associated with inflammation.

It is clear that such an improvement in the identification of situations of endometrial inflammation and reduced endometrial receptivity can have a very significant impact on the success of therapy designed to restore conditions favourable to successful embryo implantation and normal pregnancy.

The subject of the invention is therefore an *ex vivo* method for the analysis of a patient's endometrial receptivity comprising the following steps:
a. assessing the expression levels of the NALP-3 and thrombomodulin (TM) proteins with the same analytical method (or in any case methods in which the values measured can be normalised and therefore compared), in an endometrial biopsy sample and/or in an endometrial fluid sample and/or in a menstrual blood sample of said patient
b. calculating the ratio between the expression level of the NALP-3 and TM proteins [NALP-3 expression/TM expression] wherein, when said value is greater than 1, it is a marker of inflammatory alteration of the endometrium of said patient and of an altered endometrial receptivity with respect to a healthy endometrium.

As reported in the table below, a measurement of the expression levels of the NALP-3 protein and of the TM protein in an endometrial biopsy (or in endometrial fluid or in menstrual blood) makes it possible to identify up to 80% of cases of endometrial inflammation and reduced endometrial receptivity.

The expression "with the same analytical method" means that the type of assay used to determine the expression levels of NALP-3 must be the same.

This method is therefore extremely advantageous because, compared to diagnostic hysteroscopy, it is much more sensitive, is not dependent on the subjectivity of the operator, can be performed on frozen and reproducible samples, is quick, and, in contrast to methods that require a quantification of the gene expression, is based on a defined number of markers and is representative of an actual protein expression, rather than a potential protein expression, such as that provided when analysing gene expression.

In addition, as is known, in all tests in which the gene expression level is analysed, this is done on the basis of RNA, which is in fact the product of gene expression. This molecule, as is known to those skilled in the art, is an extremely delicate molecule insofar as it is susceptible to attack of the RNase, which is an extremely stable enzyme and is present in all tissues and on the basis of which the operators can also often examine the sample.

During the course of the present description, reference will be made to the ratio between the expression level of the NALP-3 and TM proteins [NALP-3 expression/TM expression] also by the acronym RIT.

For good assessment of the RIT, it is important that the same method for quantification of the expression of the NALP-3 and TM proteins is used, and therefore, in the method of the present description, the same type of assay is used to quantify the expression levels of the two proteins.

According to the method described here, when the RIT is greater than 1, this indicates endometrial inflammation and an altered endometrial receptivity. The term "altered or reduced" endometrial receptivity, as already mentioned, indicates an endometrial receptivity different from that observed in a control population formed by healthy women with at least two previous physiological pregnancies.

The authors of the present invention have confirmed that an RIT value of greater than 1 is present in 70-80% of cases of endometrial inflammation. A further rise in the sensitivity of the method can be obtained by determining the expression values of one or more inflammatory cytokines in the biopsy tissue.

In one embodiment the method as described above therefore comprises a further determination of the expression in said endometrial biopsy sample and/or endometrial fluid sample and/or menstrual blood sample of caspase-1 and/or IL-18 and/or IL-1β, at least in the samples in which the value [NALP-3 expression/TM expression] is less than or equal to 1, and wherein caspase-1 and/or IL-18 and/or IL-1β expression levels greater than those determined in control samples as defined above are indicative of inflammatory alteration of the endometrium of said patient and of reduced endometrial receptivity. In particular, the method comprises determining the expression of caspase-1 and of the cytokines IL-18 and IL-1β in said sample.

In patients with an RIT less than or equal to 1, an expression value of caspase-1 or of one or both of the aforementioned cytokines greater than the reference value observed in a control population such as that defined above (fertile women) makes it possible to identify further cases of endometrial inflammation and of altered endometrial receptivity, bringing the sensitivity of the method to approximately 90%.

According to the present description, said further determination can be carried out in step a. so as to already have the expression values of the cytokines of interest usable in cases in which the RIT is less than or equal to 1 or also for other objectives, or can be carried out after step b., and therefore for those samples with an RIT less than or equal to 1.

According to the present description therefore, an RIT with values >1 is already indicative *per se* of inflammation and altered endometrial receptivity; when the values of the RIT are ≤1 it is preferable to supplement the diagnostic procedure with an assay of capsase-1 and/or the cytokines IL-18 and/or IL-1β, which can be elevated due to other exogenous stimuli of the inflammatory type, independently of the presence of a greater activation of NALP3 or a reduced expression of TM.

As mentioned above, an increase in the expression of such further markers compared to the controls is a further indicator of inflammation and altered endometrial receptivity. The authors have found that the assay of caspase-1 seems to have a development parallel to and overlapping with that of NALP3. In principle, therefore, and in an embodiment of the invention, the determination of the expression values of NALP3 and the calculation of the RIT can be carried out by determining the expression values of caspase-1 and by using the same values in the RIT calculation instead of those for NALP3.

According to the invention, any technique known to a person skilled in the art can be used to determine the expression of the proteins of interest. A non-limiting example of techniques includes Western Blot by immunoenzymatic assays. Such immunoenzymatic assays can be carried out for example for each individual marker by means of an ELISA assay, or by means of immunoenzymatic assay of the Multiplex type.

The use of Multiplex technology, which is well known to a person skilled in the art, makes it possible to identify various desired markers concomitantly.

For the assaying of the proteins of interest by means of immunoenzymatic assay, the optical densities (O.D.) obtained by means of spectrophotometric reading are converted into ng/ml or mcg/ml using a standard reference curve.

The threshold value can be determined on the basis of the median value obtained over a control population (healthy) + 2 standard deviations.

A further subject of the invention is a kit for the analysis of endometrial receptivity comprising
a. reagents for the assessment of NALP-3 and thrombomodulin (TM) expression and, optionally,
b. reagents for the assessment of the expression of caspase-1 and/or IL-18 and/or IL-1β and control samples representative of caspase-1, and/or IL-18 and/or IL-1β expression in fertile women.

Such reagents comprise anti-NALP-3 antibodies and anti-TM monoclonal antibodies suitably arranged on ELISA or Multiplex immunoenzymatic assay supports and optionally anti-caspase-1 and/or anti-IL-18 and/or anti-IL-1β antibodies suitably arranged in ELISA or ELISA Multiplex plates.

The kit can also comprise the reagents for detecting said antibodies.

Furthermore, the kit can also comprise caspase-1 and/or IL-18 and IL-1β sample (for example aliquots thereof, suitably dosed) as a control representative of the expression in fertile women.

A further subject of the invention is a support for ELISA or Multiplex Immunoenzymatic assay comprising wells or spots coated with anti-NALP-3 antibodies and anti-TM antibodies suitably arranged and, optionally, with anti-caspase-1, IL-18, IL1β antibodies.

In the case of Multiplex assays, the various antibodies are disposed in such a way as to allow a single assay cycle in accordance with the techniques known to a person skilled in the art.

The support can therefore be a plate for ELISA, a Multiplex array for example by means of multianalytical profile techniques, known to a person skilled in the art, in which beads are coloured internally with suitable dyes so as to produce a specific pattern of spectra, the suitable biomolecules, such as the antibodies specific for the proteins of interest, being able to be conjugated to the surface of the beads so as to capture the analytes of interest.

A further subject of the present invention is the use of the kit and of the support described above for the analysis of a patient's endometrial receptivity.

This use can be implemented within the scope of medical therapy for restoring the correct endometrial receptivity.

A further subject of the present disclosure is a therapeutic method for treating patients presenting unexplained SRA or infertility, comprising
a. assessing the expression levels of the NALP-3 and thrombomodulin (TM) proteins with the same analytical method, in an endometrial biopsy sample and/or in an endometrial fluid sample and/or in a menstrual blood sample of said patient
b. calculating the ratio between the expression level of the NALP-3 and TM proteins [NALP-3 expression/TM expression] wherein, when said value is greater than 1, it is a marker of inflammatory alteration of the endometrium of said patient and of an altered endometrial receptivity with respect to a healthy endometrium, and
c. treatment with suitable therapies tailored for those patients in whom the reduced endometrial receptivity is linked to an inflammatory alteration of the endometrium identified in step b so as to restore an endometrial state that favours embryo implantation.

The therapeutic treatment detailed above can comprise or can be constituted by the administration of alpha-lipoic acid in a dosage that is therapeutically effective for a suitable period of time.

The dosage of alpha-lipoic acid and the duration of the treatment will be determined by the treating doctor based on the state of health of the patient, their weight, age, etc.

In accordance with a possible embodiment, the alpha-lipoic acid can be administered in a dosage between 400 and 1200 mg/day in one or more doses.

The duration of the treatment can be extended for the time necessary to restore physiological endometrial receptivity. By way of non-limiting example, said treatment can be extended for a period of time between two and four months.

During the treatment, the assessment of the RIT and of any further markers as described above can be repeated once or more.

The present disclosure also relates to alpha-lipoic acid for use in the treatment of patients suffering from endometrial inflammation and altered endometrial receptivity. This pathological state can be determined by means of the diagnostic method described herein.

Alpha-lipoic acid, optionally in combination with further pharmaceutical products or active ingredients, for the use as stated above, can be administered in a dosage between 400 and 1200 mg/day in one or more doses for a period between one and six months.

In one embodiment the treatment can be a treatment with a dosage of alpha-lipoic acid of approximately 400 mg twice a day, for a period of approximately three months.

The treatment can comprise the concomitant or sequential administration of further active ingredients in combination with the alpha-lipoic acid.

The treatment can be provided for the length of time necessary to re-establish a physiological endometrial receptivity. By way of non-limiting example, said treatment can be provided for a period between two and four months.

The experimental data collected by the authors of the invention will be reported below.

All of the assays were performed on patients who had given their informed consent for the treatment of the removed tissues.

The method and kit of the invention were used on patients who had given their informed consent for analysis of the removed biological sample.

### EXAMPLES

### 1. Diagnostic hysteroscopy and biopsy.

Diagnostic hysteroscopy with biopsy was performed in an ambulatory environment after having confirmed the absence of certain conditions. In particular: absence of current pregnancy and absence of infection under vaginal culture test and examination of the endometrium and seminal liquid (Chlamydia Trachomatis, mycoplasmas, Neisseria gonorrhoea, yeasts, Trichomonas, Streptrococco agalacitiae B, Listeria Monocytogenes, Gardnerella, other gram positive and gram negative microorganisms). The hysteroscopy was performed for the period of the implantation window (days 19-24 of the cycle) and included assay on the same day of progesterone and choriogonadotropin subunit beta (β-hCG).

The hysteroscopy picture of endometritis is defined by: diffuse or focal endometrial hyperaemia ("strawberry-like" aspect), combined with oedema of the mucous and with microlipi (<1mm).

The endometrial biopsy was performed using a Novak cannula (3 mm size) inserted with targeted guidance through the cervical channel, with particular attention and avoidance of contact of the cannula with the vaginal channel. Once collected, the sample was washed in sterile physiological solution and stored at -80°C.

### 2. Assay of NALP-3 and TM

For assay of NALP-3 and TM, the western-blot method was used. For the analysis, the total lysed cells obtained from endometrial biopsy were separated by means of 10% acrylamide gel electrophoresis. After electrophoresis, the isolated proteins were transferred to a nitrocellulose membrane and incubated at 4°C with a specific primary antibody (anti-NALP3, 1:500, ThermoFisher Scientific, Rockford, IL, USA or anti-TM, 1:500, Cell Signaling Technology, Danvers, MA 01923, USA). The membranes were then washed with PBST and incubated with specific secondary antibody (1:2000). The secondary antibody, once bound, was identified by means of chemiluminescence. The protein bands obtained were acquired and analysed using the Gel Doc 200 analysis system and quantified by means of specific software (Quantity One Quantitation Software, BioRad). The protein levels (NALP-3 or TM) were calculated in relation to the constant level of a protein of molecular weight 42-kDa (internal β-actin control, Sigma-Aldrich, St Louis, MO, USA).

Similar results were obtained by means of ELISA assay.

### 3. Assay of caspase-1, IL-18 and IL-1β.

The levels of caspase-1, IL-18 and IL-1β were assayed in the lysed cells obtained from endometrial biopsy by means of immunoenzymatic assay (ELISA) in accordance with the instructions provided on the kit (USCN Life Science Inc. and Cloud-Clone Corp. Houston, TX, USA). The samples or the standards (100 µl) were briefly plated in wells containing the human anticlonal antibody anti-caspases 1, IL-18, and IL-1β. After 2 hours of incubation at 37°C, the wells were washed and incubated with a specific secondary polyclonal antibody bound to the peroxidase enzyme. The substrate tetramethyl benzidine was then added so as to initiate a chemical reaction producing a coloured product, the intensity of which is directly proportional to the amount of protein bound in the initial step. The plates were read using a Titertek Multiscan plus Mk II plate reader (ICN Flow Laboratories, Irvine, CA), measurement of absorbance at a wavelength of 450 nm.

### 4. Study of endometrial receptivity using the method of the invention

Patient group: women with SRA and women with unexplained infertility. The women, after having given informed consent, underwent a hysteroscopic biopsy during the window of implantation (days 19-24 of the cycle, having been informed beforehand to avoid falling pregnant during the month of the hysteroscopy and following a negative pregnancy test), using 3 mm Noval curette (Cooper Surgical Inc.). The biopsies were subjected to histological and functional examination. The diagnosis of chronic endometritis was performed by means of histological examination.

The concentration of NALP-3, TM, caspases-1, IL-1β and IL-18 was quantified using the ELISA immunoenzymatic method.

The lysed tissue was added to the wells, which had been covered beforehand with anti-NALP-3, anti-TM, and anti-caspase-1, anti-IL-Iβ and anti-IL-18 monoclonal antibodies.

After 2 hours of incubation, the wells were washed and incubated with specific secondary antibody conjugated with peroxidase. The substrate solution was then added (3,3',5,5'-tetramethyl benzidine, TMB) and the colour developed in each well, in a manner proportional to the amount of present proteins under examination, and this was measured by way of spectrophotometry using a plate reader (Plate Reader DAS; DAS srl, Italy) at a wavelength of 450 nm.

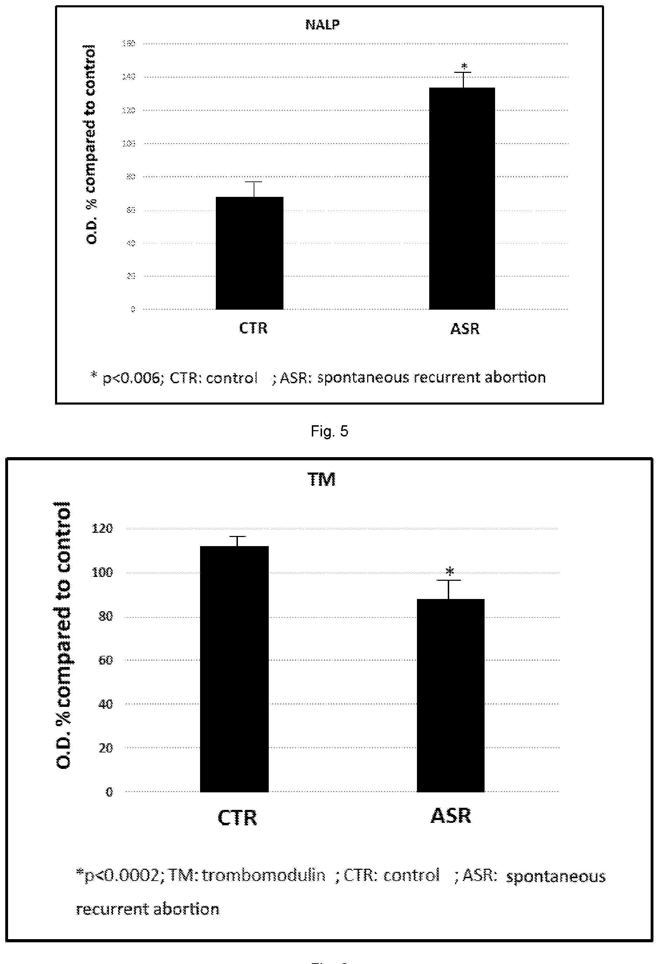

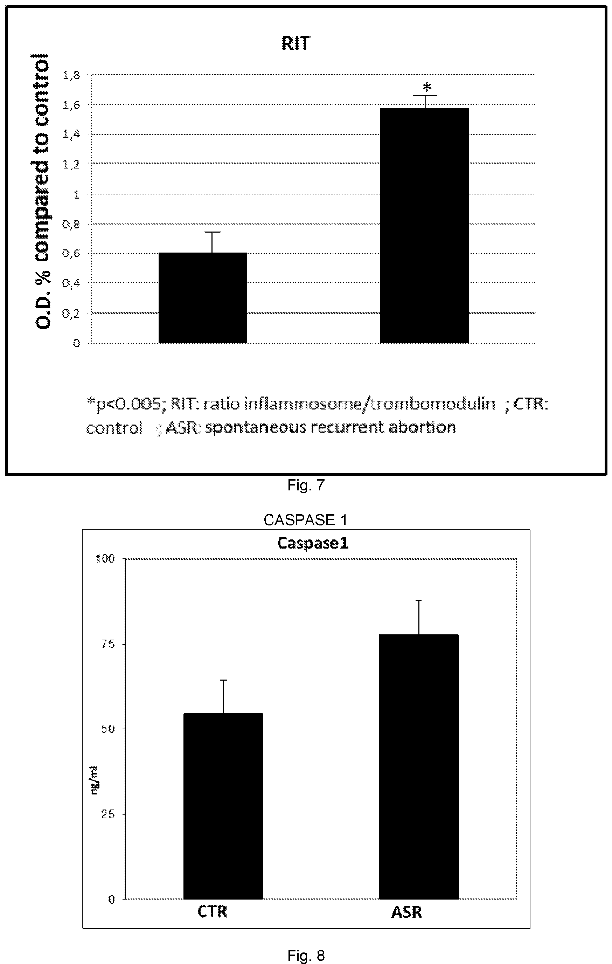

It was possible to use one or more recombinant proteins; NALP-3, TM, and caspases-1, IL-1β and IL-18 were used to form a standard curve suitable for use for determining the protein expression expressed in pg/ml in each sample analysed. Table 1 below shows the levels of NALP-3 and TM measured in the samples used by means of western-blot analysis. (Figures 5, 6 and 7)

**Table 1.**

| **CTR** | **AR** | **AR** |
|---|---|---|
| **O.D. (optical density)** | **O.D.** | **O.D.** |
| 2081 | 3230 | 4122 |
| 2780 | 3799 | 2998 |
| 2885 | 5781 | 2783 |
| 2514 | 4996 | 1935 |
| 3600 | 3609 | 3817 |
| 2466 | 3400 | 7526 |
| 3097 | 4128 | 4990 |
| 1734 | 4696 | 3463 |
| 1125 | 5360 | 2526 |
| 2078 | 5640 | 3871 |
| 2650 | 4669 | 3063 |
| 2901 | 6892 | 4678 |
| 2456 | 4758 | 3097 |
| 3590 | 5404 | 2019 |
| 2503 | 4833 | 5371 |
| 3100 | 5044 | 5911 |
| 1686 | 4921 | 3961 |
| 1135 | 4468 | 2535 |
| | 8549 | 5311 |
| | 4081 | 2734 |
| | 3524 | 3307 |
| | 3856 | 5275 |
| | 5869 | 3824 |
| | 4670 | 3313 |
| | 6869 | |

Table 2 below shows the values calculated for the marker TM dosed by way of western blot.

**Table 2**

| **CTR** | **AR** | **AR** |
|---|---|---|
| **O.D. (optical density)** | **O.D.** | **O.D.** |
| 1512 | 613 | 645 |
| 1685 | 1338 | 1302 |
| 1249 | 1171 | 1230 |
| 1404 | 1235 | 1790 |
| 1498 | 1311 | 1320 |
| 1720 | 1350 | 1250 |
| 1198 | 1070 | 1102 |
| 1490 | 1052 | 998 |
| 1500 | 1003 | 1050 |
| 1698 | 1027 | 1002 |
| 1340 | 1105 | 1230 |
| 1235 | 1215 | 1102 |

Table 3 below shows the values calculated for the marker caspase-1 measured by way of immunoenzymatic assay (ELISA).

**Table 3**

| **CTR** | **AR** |
|---|---|
| ng/ml | ng/ml |
| 50.8 | 116 |
| 52 | 60 |
| 45.4 | 72 |
| 50 | 82 |
| 87.3 | 46 |
| 56 | 72 |
| 41.8 | 62 |
| 53 | 110 |
| 51.5 | 41.6 |
| 45.2 | 42.8 |
| 48 | 55.2 |
| 86.8 | 46 |
| 57.5 | 60 |
| 43.2 | 64 |
| 47.9 | 47 |
| 54.2 | 87.3 |
| 43.1 | 65.6 |
| 46 | 67.1 |
| 84 | 82.3 |
| 60.1 | 64 |
| 45.2 | 100 |
| | 59 |
| | 66.2 |
| | 74.2 |
| | 69 |
| | 292 |
| | 98.2 |

Table 4 below shows the values calculated for the marker IL 1β measured by way of immunoenzymatic assay (ELISA).

**Table 4**

| **CTR** | **AR** | **AR** |
|---|---|---|
| ng/ml | ng/ml | ng/ml |
| 82 | 194 | 192 |
| 81.4 | 180 | 130 |
| 106 | 160 | 131 |
| 88.8 | 372 | 208 |
| 91.2 | 142 | 112 |
| 104 | 158 | 85 |
| 67.2 | 156 | 146.6 |
| 79 | 134 | 109 |
| 84.5 | 144 | 141 |
| 109.5 | 156 | 120 |
| 84.3 | 152 | 150 |
| 95.6 | 210 | 73.6 |
| 97.9 | 138 | |
| 66.9 | 130 | |

Table 5 below shows the values calculated for the marker IL 18 measured by way of immunoenzymatic assay (ELISA).

**Table 5**

| **CTR** | **AR** | **AR** |
|---|---|---|
| microg/ml | microg/ml | microg/ml |
| 3 | 2.1 | 5.9 |
| 1.4 | 1.8 | 13.5 |
| 2.1 | 4.8 | 4.8 |
| 1.6 | 3.9 | 4.1 |
| 5.2 | 3 | 7.5 |
| 4.04 | 3.5 | 6.2 |
| 4.9 | 2.9 | 8.2 |
| 2.8 | 4.2 | 7.4 |
| 1.8 | 3.2 | 12.01 |
| 2.5 | 3.5 | 4.5 |
| 1.4 | 6.2 | |
| 5.9 | 3.5 | |
| 3.8 | 3.1 | |
| 5.2 | 4 | |
| 2.4 | 3.9 | |
| 1.9 | 6.8 | |
| 2.6 | 7.6 | |

### 5. Treatment of AR with alpha-lipoic acid

Alpha-lipoic acid (ALA) is a commercially available molecule with wide-ranging dosages (normally 100-800 mg/dose) with antioxidant properties, and is widely used in clinical practice.

Approximately 30 women (who had given their consent) suffering from idiopathic abortion were treated for three months with daily doses of 400 mg of alpha-lipoic acid. Both before therapy and at the end thereof, the inflammasomes and the values of NALP-3 and TM from endometrial biopsies were assessed. The preliminary data obtained from this first group of patients shows a normalisation of the activity of NALP-3 and TM.

## Claims

1. An in vitro method for the analysis of a patient's endometrial receptivity comprising the following steps
a. assessing the expression levels of the NALP-3 and Thrombomodulin (TM) proteins with the same analytical method, in an endometrial biopsy sample and/or in an endometrial fluid sample and/or in a menstrual blood sample of said patient
B. calculating the ratio between the expression level of the NALP-3 and TM proteins [NALP-3 expression/TM expression] wherein, when said value is greater than 1, it is a marker of inflammatory alteration of the endometrium of said patient and of an altered endometrial receptivity with respect to a healthy endometrium.

2. The method as claimed in claim 1 further comprising assessing the expression levels of caspase-1 and/or IL-18 and/or IL-1β in said endometrial biopsy specimen and/or endometrial fluid sample and/or menstrual blood sample at least in the biopsy specimens in which the value [NALP-3 expression/TM expression] is less than or equal to 1, and wherein IL-18 and IL-1β expression values greater than those determined in control samples representative of fertile women, are indicative of inflammatory alteration of the endometrium of said patient and of reduced endometrial receptivity compared to an endometrial of fertile women even in patients with a value (NALP-3 expression/TM expression] less than or equal to 1.

3. The method according to claim 2, wherein said further assessment is carried out in step a. or after step b..

4. The method according to anyone of claims 1 to 3, wherein said expression assesments are performed by Western Blot and/or by immunoenzymatic assay.

5. The method of claim 4 wherein said immunoenzymatic assay is an ELISA assay or a multiplex-type immunoenzymatic assay.

6. A kit for the analysis of endometrial receptivity of a patient comprising
a. reagents for the assessment of NALP-3 and Thrombomodulin (TM) expression and, optionally,
b. reagents for the assessment of the expression of caspase-1 and/or IL-18 and IL-1β and control samples representative of caspase-1, IL-18 and IL-1β expression in fertile women wherein said reagents comprise anti-NALP-3 antibodies and anti-TM antibodies suitably arranged in ELISA or Multiplex immunoenzymatic plates and optionally anti-caspase-1 and/or anti-IL-18 and/or anti-IL-1β antibodies suitably arranged in ELISA or ELISA Multiplex plates.

7. The kit according to claim 6 further comprising caspase-1 and/or IL-18 and IL-1β aliquots suitably dosed as a control representative of the expression of said markers in fertile women.

8. A support for ELISA or Multiplex Immunoenzymatic Assay comprising anti-NALP-3 antibodies and anti-TM antibodies suitably arranged.

9. A support according to claim 8 further comprising in wells an anti-caspase-1 and/or anti-IL-18 antibody and/or antiL-1β antibodies.

10. The use of the kit according to claims 6 or 7 or of the support according to claims 8 or 9 for the analysis of a patient's endometrial receptivity.

## Patentansprüche

1. In-Vitro-Verfahren zur Analyse der endometrialen Aufnahmefähigkeit einer Patientin umfassend die folgenden Schritte
a. Bestimmen der Expressionsniveaus der NALP-3- und Thrombomodulin (TM)-Proteine mit demselben analytischen Verfahren in einer endometrialen Biopsieprobe und/oder in einer endometrialen Flüssigkeitsprobe und/oder in einer Menstruationsblutprobe der Patientin
B. Berechnen des Verhältnisses zwischen dem Expressionsniveau der NALP-3- und TM-Proteine [NALP-3-Expression/TM-Expression], wobei, wenn der Wert größer als 1 ist, dies ein Marker für eine entzündliche Veränderung des Endometriums der Patientin und für eine veränderte endometriale Aufnahmefähigkeit gegenüber einem gesunden Endometrium ist.

2. Verfahren gemäß Anspruch 1, ferner umfassend das Bestimmen der Expressionsniveaus von Caspase-1 und/oder IL-18 und/oder IL-1β der endometrialen Biopsieprobe und/oder der endometrialen Flüssigkeitsprobe und/oder der Menstruationsblutprobe zumindest in den Biopsieproben, in denen der Wert [NALP-3-Expression/TM-Expression] kleiner oder gleich 1 ist, und wobei IL-18- und IL-1β-Expressionswerte, die größer als diejenigen sind, die in Kontrollproben, die für fruchtbare Frauen repräsentativ sind, bestimmt wurden, auf eine entzündliche Veränderung des Endometriums der Patientin und auf eine verringerte endometriale Aufnahmefähigkeit im Vergleich zu einem Endometrium von fruchtbaren Frauen hinweisen, selbst bei Patientinnen mit einem Wert [NALP-3-Expression/TM-Expression] kleiner oder gleich 1.

3. Verfahren gemäß Anspruch 2, wobei das weitere Bestimmen in Schritt a oder nach Schritt b ausgeführt wird.

4. Verfahren gemäß einem der Ansprüche 1 bis 3, wobei die Expressionsbestimmungen durch Western Blot und/oder durch einen immunoenzymatischen Test durchgeführt werden.

5. Verfahren gemäß Anspruch 4, wobei der immunoenzymatische Test ein ELISA-Test oder ein multiplexartiger immunoenzymatischer Test ist.

6. Set zur Analyse der endometrialen Aufnahmefähigkeit einer Patientin umfassend
a. Reagenzien zum Bestimmen der NALP-3- und Thrombomodulin (TM)-Expression und, optional,
b. Reagenzien zum Bestimmen der Expression von Caspase-1 und/oder IL-18 und IL-1β und Kontrollproben, die für die Expression von Caspase-1, IL-18 und IL-1β bei fruchtbaren Frauen repräsentativ sind, wobei die Reagenzien Anti-NALP-3-Antikörper und Anti-TM-Antikörper, die in geeigneter Weise in ELISA- oder multiplex-immunoenzymatischen Platten angeordnet sind, und optional Anti-Caspase-1- und/oder Anti-IL-18- und/oder Antil-IL-1β-Antikörper, die in geeigneter Weise in ELISA- oder ELISA-Multiplexplatten angeordnet sind, umfassen.

7. Set gemäß Anspruch 6, ferner umfassend Caspase-1- und/oder IL-18- und IL-1β-Teilproben, die in geeigneter Weise dosiert sind, als eine Kontrolle, die für die Expression der Marker bei fruchtbaren Frauen repräsentativ sind.

8. Träger für einen ELISA- einen multiplex-immunoenzymatischen Test, umfassend Anti-NALP-3-Antikörper und Anti-TM-Antikörper, die geeignet angeordnet sind.

9. Träger gemäß Anspruch 8, ferner umfassend in Senken Anti-Caspase-1- und/oder Anti-I L-18-Antikörper und/oder Anti-IL-1β-Antikörper.

10. Verwendung des Sets gemäß Anspruch 6 oder 7 oder des Trägers gemäß Anspruch 8 oder 9 für die Analyse der endometrialen Aufnahmefähigkeit einer Patientin.

## Revendications

1. Procédé in vitro pour l'analyse de la réceptivité endométriale d'une patiente, comprenant les étapes suivantes :
a. la détermination des niveaux d'expression des protéines NALP-3 et thrombomoduline (TM) par le même procédé analytique, dans un échantillon de biopsie endométriale et/ou dans un échantillon de fluide endométrial et/ou dans un échantillon de sang menstruel de ladite patiente,
b. calcul du rapport entre le niveau d'expression des protéines NALP-3 et TM [expression de NALP-3/expression de TM], dans lequel, quand ladite valeur est supérieure à 1, ceci constitue un marqueur d'une altération inflammatoire de l'endomètre de ladite patiente et d'une réceptivité endométriale altérée par rapport à un endomètre sain.

2. Procédé selon la revendication 1, comprenant en outre la détermination des niveaux d'expression de caspase-1 et/ou d'IL-18 et/ou d'IL-Iβ dans ledit échantillon de biopsie endométriale et/ou ledit échantillon de fluide endométrial et/ou ledit échantillon de sang menstruel au moins dans les échantillons de biopsie dans lesquels la valeur [expression de NALP-3/expression de TM] est inférieure ou égale à 1, et dans lequel des valeurs d'expression d'IL-18 et d'IL-Iβ supérieures à celles déterminées dans des échantillons témoins représentatifs d'une femme fertile sont indicatives d'une altération inflammatoire de l'endomètre de ladite patiente et d'une réceptivité endométriale réduite en comparaison avec l'endomètre d'une femme fertile même chez les patientes ayant une valeur [expression de NALP-3/expression de TM] inférieure ou égale à 1.

3. Procédé selon la revendication 2, dans lequel ladite détermination supplémentaire est effectuée dans l'étape a. ou après l'étape b.

4. Procédé selon l'une quelconque des revendications 1 à 3, dans lequel lesdites déterminations d'expression sont effectuées par transfert Western et/ou par dosage immuno-enzymatique.

5. Procédé selon la revendication 4, dans lequel ledit dosage immuno-enzymatique est un dosage ELISA ou un dosage immuno-enzymatique de type multiplex.

6. Trousse pour l'analyse de la réceptivité endométriale d'une patiente, comprenant
a. des réactifs pour la détermination de l'expression de NALP-3 et de thrombomoduline (TM), et éventuellement
b. des réactifs pour la détermination de l'expression de caspase-1 et/ou d'IL-18 et d'IL-Iβ et des échantillons témoins représentatifs de l'expression de caspase-1, d'IL-18 et d'IL-Iβ chez une femme fertile,
dans laquelle lesdits réactifs comprennent des anticorps anti-NALP-3 et des anticorps anti-TM convenablement disposés dans des plaques ELISA ou immuno-enzymatiques multiplex et éventuellement des anticorps anti-caspase-1 et/ou anti-IL-18 et/ou anti-IL-Iβ convenablement disposés dans des plaques ELISA ou multiplex ELISA.

7. Trousse selon la revendication 6, comprenant en outre des aliquotes de caspase-1 et/ou d'IL-18 et d'IL-Iβ convenablement dosées en tant que témoin représentatif de l'expression desdits marqueurs chez une femme fertile.

8. Support pour dosage ELISA ou immuno-enzymatique multiplex comprenant des anticorps anti-NALP-3 et des anticorps anti-TM convenablement disposés.

9. Support selon la revendication 8, comprenant en outre, dans des puits, un anticorps anti-caspase-1 et/ou un anticorps anti-IL-18 et/ou des anticorps anti-IL-1β.

10. Utilisation de la trousse selon la revendication 6 ou 7 ou du support selon la revendication 8 ou 9 pour l'analyse de la réceptivité endométriale d'une patiente.
